# EUROPÄISCHE PATENTSCHRIFT

(11) **EP 0 775 150 B1**
(45) Veröffentlichungstag und Bekanntmachung des Hinweises auf die Patenterteilung: **28.04.1999**
(21) Anmeldenummer: 95907641.5
(22) Anmeldetag: 03.02.1995
(51) Int. Cl.: C07H 1/08, C12N 15/10, C12P 19/34

(54) **VERFAHREN ZUR ABREICHERUNG ODER ENTFERNUNG VON ENDOTOXINEN**
ENDOTOXIN REDUCTION OR REMOVAL PROCESS
PROCEDE DE REDUCTION OU D'ELIMINATION D'ENDOTOXINES

(30) Priorität: 07.02.1994 DE 4403692; 25.06.1994 DE 4422291; 01.09.1994 DE 4431125; 14.09.1994 DE 4432654
(43) Veröffentlichungstag der Anmeldung: 28.05.1997
(73) Patentinhaber: QIAGEN GmbH, 40724 Hilden (DE)
(72) Erfinder: COLPAN, Metin, D-45219 Essen (DE); MORITZ, Peter, D-50169 Kerpen (DE); SCHORR, Joachim, D-40231 Düsseldorf (DE)
(74) Vertreter: Meyers, Hans-Wilhelm, Dr.Dipl.-Chem.
(86) Internationale Anmeldenummer: EP9500391
(87) Internationale Veröffentlichungsnummer: WO9521179

(56) Entgegenhaltungen:
- WO-A-93/11221
- US-A- 4 997 932
- DATABASE WPI Section Ch, Week 8310 Derwent Publications Ltd., London, GB; Class A96, AN 83-23220 & JP,A,58 013 519 (SEIKAGAKU KOGYO KK) , 26.Januar 1983

## Beschreibung

Gegenstand der vorliegenden Erfindung ist ein Verfahren zur Abreicherung oder Entfernung von Endotoxinen aus für den therapeutischen Einsatz vorgesehenen Wirkstoffe enthaltenden Präparaten, die gen- und/oder biotechnologisch aus natürlichen Quellen gewonnen werden, durch Behandlung mit chromatographischem Material, sowie eine entsprechende Verwendung, eine wäßrige Lösung zur Rückführung des Verfahrens und eine Zusammenstellung enthaltend Elemente zur Durchführung des Verfahrens.

Molekularbiologische Verfahren gewinnen zunehmend Bedeutung bei der Herstellung von Arzneimitteln. Dazu gehören zum einen die klassischen gentechnologischen Verfahren zur Herstellung von Arzneimitteln und in zunehmenden Maße auch die sogenannte Gentherapie, bei der Nucleinsäuren in das Genom zu behandelnder Spezies eingeschleust werden. Dabei bekommt die Entfernung von Endotoxinen entscheidende Bedeutung.

Um beispielsweise Plasmid DNA im präparativen Maßstab herzustellen, ist es notwendig die Plasmide mit Hilfe sogenannter Wirtszellen zu vermehren. Hierbei handelt es sich im allgemeinen um gram-negative Enterobakterien, wie beispielsweise Mutanten von E.coli K-12. Gram-negative Bakterien weisen eine Zellwand auf, die von einer äußeren Membran umgeben ist. Auf dieser Membran befinden sich sogenannte Lipopolysaccharide (LPS), die auch unter dem Begriff Endotoxine bekannt sind. Die Endotoxine sind beispielsweise verantwortlich für die typischen Erscheinungen, die mit einer bakteriellen Intoxikation verbunden sind, wie entzündlichen Reaktionen und Fieber sowie endotoxischem Schock.

Wie in der WO-A-95/21177 und DE-A-44 22 291 erwähnt ist, wird innerhalb der in vivo und ex vivo Gentherapie Plasmid DNA zur Behandlung von genetisch bedingten Erkrankungen wie beispielsweise der cystischen Fibrose, aber auch zur Behandlung von Krebs oder Hämophilie bzw. zur Immunisierung gegen Infektionskrankheiten verwendet (TIBTECH, Special Issue: Gene Therapy Therapeutic Strategy and Commercial Prospects, Mai 1993, Vol. 11, Nr. 5 (112)). Dabei ist es von entscheidender Bedeutung, daß die verabreichte DNA keine Nebenreaktionen wie beispielsweise inflammatorische oder nekrotische Reaktionen hervorruft. Es muß also sichergestellt sein, daß die für diese Art der Behandlung verwendete DNA nicht mit Endotoxinen kontaminiert ist.

Auch gentechnologisch oder biotechnologisch hergestellte Präparate können Endotoxine enthalten. Wichtig ist daher die Entfernung oder Abreicherung der Endotoxine unter physiologisch unbedenkliche Mengen.

Die zur Zeit bekannten Reinigungsverfahren, beispielsweise für Plasmid DNA aus gram-negativen Bakterien, können die Endotoxine nicht vollständig aus der Plasmid DNA entfernen. Zu diesen Methoden gehören zum Beispiel Cäsiumchlorid Gradienten Zentrifugation oder Anionenaustauscher-Chromatographie.

Die Cäsiumchlorid-Gradientenzentrifugation beruht darauf, daß verschieden große DNA-Moleküle eine unterschiedliche Wanderungsgeschwindigkeit in einem Salzgradienten aufweisen.

Lipopolysaccharide zeigen jedoch das gleich Migrationsverhalten im Dichtegradienten wie die DNA und sind somit nicht effektiv von der DNA zu trennen.

Verglichen mit der Anionenaustauscher-Chromatographie ist die Cäsiumchlorid-Gradientenzentrifugation sehr zeitaufwendig und verwendet eine Reihe von toxischen Substanzen, wie beispielsweise Ethidiumbromid. Weiterhin muß durch eine zusätzliche Dialyse Cäsiumchlorid entfernt werden.

Aida und Pabst in "Removal of endotoxin from Protein solutions by phase separation using Triton X 114", J. Immunol. Methods 132, 191 - 195 (1990) schlagen eine Methode vor, um Endotoxine mittels Triton® X 114 Extraktion aus Protein Lösungen zu entfernen. Hierbei wird die zu behandelnde Lösung mit dem Detergenz Triton® X 114 versetzt. Die proteinhaltige, wäßrige Phase wird nach Inkubation und Zentrifugation abgenommen und das Endotoxin befreite oder an Endotoxin abgereicherte Protein präzipitiert Wie in der WO 95/21177 bereits vorgeschlagen wird, kann dieses Verfahren auch zur Extraktion von Endotoxinen aus DNA-Lösungen angewandt werden. Diese Methode ist jedoch durch einen recht hohen Arbeitsaufwand gekennzeichnet und wird erst nach der Isolierung der DNA eingesetzt. Für die Reinigung von DNA-Mengen im präparativen, insbesondere im industriellen Maßstab, ist dieses Verfahren weniger geeignet.

Auch US-A-5 136 026 beschreibt ein Verfahren zur Entfernung von Toxinen aus Proteinlösungen, insbesondere von Lipocortinen.

Das der Erfindung zugrundeliegende technische Problem besteht nunmehr darin ein Verfahren anzugeben, mit dem es gelingt endotoxinfreie oder an Endotoxinen abgereicherte Präparate herzustellen, die für den therapeutischen Einsatz vorgesehene Wirkstoffe enthalten und aus gen- und/oder biotechnologischen Quellen stammen. Die oben beschriebenen Nachteile soll das erfindungsgemäße Verfahren vermeiden. Dabei soll die Aufreinigung der DNA und die Abtrennung oder Abreicherung der Endotoxine im selben Verfahren oder Verfahrensschritt erfolgen.

Das technische Problem wird gelöst durch ein Verfahren mit den Merkmalen des Anspruchs 1. Die Unteransprüche 2 bis 7 betreffen bevorzugte Ausführungsformen des erfindungsgemäßen Verfahrens. Anspruch 8 betrifft die Verwendung eines Anionenaustauschers zur Abreicherung oder Entfernung von Endotoxinen aus nucleinsäurehaltigen Präparaten.

Beim erfindungsgemäßen Verfahren werden zunächst die natürlichen Quellen, aus denen die für den therapeutischen Einsatz vorgesehenen Wirkstoffe enthaltenden Präparaten gen- oder biotechnologisch gewonnen werden, aufgeschlossen. Der Aufschluß erfolgt dabei vorzugsweise nach an sich bekannten Verfahren wie beispielsweise der alkalischen Lyse, aber auch bei anderen Lyseverfahren, wie beispielsweise der Anwendung von hohem Druck (French Press), der Boiling Lyse oder der Verwendung von Detergenzien oder Lysozym. Gegebenenfalls wird das mit der alkalischen Lyse gewonnene Material durch Zentrifugations- oder Filtrationsschritte von groben Zellbruchstücken befreit.

Vor der eigentlichen Reinigungsprozedur, beispielsweise mittels üblicher Anionenaustauscher-Chromacographie, wird erfindungsgemäß das "cleared Lysate" (cL), das zum Beispiel durch die alkalische Lyse erhalten wurde, anschließend gemäß der in der PCT/EP 95/00392 vorgeschlagenen Verfahrensweise filtriert wird und dann mit Salz/Detergenzien-Kombinationen vorinkubiert.

Die deutsche Patentanmeldung PCT/EP 95/00392 schlägt ein Verfahren und eine Vorrichtung zur Isolierung von Zellinhaltsstoffen, wie Nucleinsäuren, aus natürlichen Quellen vor. Das dort beschriebene Filtrationsverfahren zur Präparation von Nucleinsäuren geht davon aus, daß die nucleinsäurehaltigen Quellen aufgeschlossen werden, der Aufschluß für einen Zeitraum ruht, der resultierende Aufschluß eine Filterschicht aus Glas, Silicagel, Aluminiumoxid oder geschütteter Diatomeenerde oder verwebter oder verklebter Vliese aus Glasfaser und Silicagel sowie Cellulose, Papier, gepreßtem Papier, Vliesen aus Papier sowie Partikel oder Schichten, Membranen oder Kunststoffe, wie beispielsweise non woven fabrics, basierend auf Polypropylen, passiert, danach die aus der Filterschicht austretende Fraktion aufgefangen wird und anschließend die Nucleinsäure aus der aufgefangenen Fraktion weiter aufgearbeitet wird. Dabei können die Filterschichten so modifiziert sein, daß keine Affinität zur Nucleinsäure besteht, insbesondere durch Hydroxylgruppen tragende Mineralien oder beschichtete Mineralien, insbesondere Diol-Silicagel, Diol-Diatomeenerde und/oder Diol-Perlite. Dies kann unter Bedingungen erfclgen, bei denen Silicagel keine Affinität zu Nucleinsäuren besitzt. Eine bevorzugte Vorrichtung zur Durchführung des in PCT/EP 95/00392 vorgeschlagenen Verfahrens weist vorzugsweise einen zylindrischen Hohlkörper auf, in dem eine Filtrationseinrichtung angeordnet ist. Die Filterschicht besteht insbesondere aus einer geschütteten Lage Diatomeenerde, deren Partikelgröße im Bereich von 5 µm bis 500 µm bei einer gesamten Filterschichtdicke von 0,1 bis 200 mm beträgt. Es kann dann vorteilhaft sein, eine zusätzliche Schicht in dem Hohlkörper anzuordnen und zwar oberhalb und/oder unterhalb der Diatomeenerdeschicht, welche ein vorzeitiges Einbringen der zu filtrierenden Lösung in den Filter oder das Austreten der Lösung aus der vorgeschlagenen Vorrichtung verhindert.

Die in der PCT/EP 95/00392 vorgeschlagene Vorrichtung ist vorteilhafterweise mit weiteren zur Nucleinsäurepräparation brauchbaren Instrumenten kombinierbar, wie sie beispielsweise in der 41 39 664 A1 offenbart sind.

Die 41 27 276 A1 offenbart Anionenaustauscher, die in eine Membran eingebettet sind (3M Empore®-Membran). Diese Systeme sind unter der Bezeichnung QIAWELL® erhältlich.

Als Inkubationslösungen kommen beispielsweise Salzlösungen von Natriumchlorid, Kaliumchlorid, Guanidiniumhydrochlorid, Natriumperchlorat und anderen chaotropen Salzen in Frage.

Als Detergenzien können insbesondere solche wie NP40, Tween® 80, Tween® 20, Triton® X 100, Triton® X 114, Syperonic® F-68 oder andere nicht ionische Detergenzien verwendet werden. Die Detergenzien liegen vorzugsweise in Konzentrationen von 0,1 % bis 30 % vor. Die Salzlösungen weisen üblicherweise Ionenstärken auf, die einer Ionenstärke einer 0,1 - 2,0 M NaCl-Lösung entsprechen.

Weiterhin kann das filtrierte Lysat auch mit einem affinitäts-chromatographischen Material inkubiert werden. Hierbei kommt insbesondere ein an Silicagel gebundener Celatbildner in Betracht. Bewährt haben sich Affinitätsmaterialien, wie mit NTA (Nitrilotetraacetat) oder IDA (Iminodiacetat) modifizierte Silicaoberflächen. Auf diesem Affinitätsträger sind beispielsweise Nickelionen komplex gebunden, welche über weitere Koordinationsstellen mit in der Seitenkette stickstoffhaltigen Aminosäureresten in Proteinen in Wechselwirkung treten können. Das filtrierte Lysat kann insbesondere mit Ni-NTA-Chromatographiematerial auf Kieselgelbasis inkubiert werden. Das Chromatographiematerial kann beispielsweise nach der erfolgten Inkubation abzentrifugiert werden, sofern im Batch-Verfahren gearbeitet wurde, und der Überstand kann über Anionenaustauscher oder andere Materialien weiter gereinigt werden. Neben dem Batch-Verfahren kann aber auch in Säulen gearbeitet werden, sofern die Beschaffenheit der Probe dies ermöglicht.

Der Anionenaustauscher ist vorzugsweise ein Material auf Basis von polymerem anorganischem Trägermaterial, wie Acryl, Dextran, Agarose oder Kombinationen davon, wobei die hier an den Anionenaustauscher gebundenen Gruppen eine Oberflächenladung von 1 bis 4 µM/m² Trägeroberfläche entsprechend 0,5 bis 500 µM/ml aufweisen. Vorzugsweise kann das in der P 44 03 692 vorgeschlagene chromatographische Trägermaterial als modifiziertes poröses oder nicht poröses anorganisches und/oder organisches Material verwendet werden. Weiterhin können Anionaustauschermaterialien, wie zum Beispiel QIAGEN®, DEAE-Sepharose®, Q-Sepharose®, DEAE-Sephadex®, Poros 20 M/P und/oder Poros 50 M/P nach der von Endotoxin befreienden Behandlung verwendet werden.

Im Anschluß an die erfindungsgemäße Verfahrensweise zur Entfernung oder Abreicherung von Endotoxinen kann die DNA auch über anorganische Materialien, wie Silicagel, Diatomeenerde, Glas, Aluminiumoxid, Titanoxid, Hydroxylapatit oder über anorganische Materialien, wie Agarose, Dextran, Acrylamid, Polystyrolharze sowie Copolymere aus den monomeren Bausteinen der genannten Monomere, aufgereinigt werden.

Mit dem erfindungsgemäßen Verfahren lassen sich insbesondere Wirkstoffe wie Nucleinsäure zum Beispiel Plasmid-DNA endotoxinfrei erhalten. Desweiteren können Nucleinsäuren wie RNA, YACs oder genomische DNA der Größen von 6 bp bis 1.000 kbp endotoxinfrei erhalten werden. Als natürliche Quellen, aus denen beispielsweise die Nucleinsäuren oder die Wirkstoffe für den therapeutischen Einsatz gewonnen werden, sind beispielsweise Zellen, Zellorganellen, Gewebe oder Mikroorganismen zu nennen.

Desweiteren können Proteinlösungen oder Viruspartikel, wie beispielsweise Adenoviren, AAV oder Retroviren, durch die erfindungsgemäße Verfahrensweise von Endotoxinen befreit oder im Endotoxingehalt abgereichert werden.

Die Vorinkubation des "cleared Lysate" erfolgt vorzugsweise mit Salzlösungen, die Alkalihalogenidsalze, nichtionische Tenside und Puffersubstanzen enthalten. Die Alkalihalogenidkonzentration entspricht hierbei einer Ionenstärke wie einer Konzentration, die 0,1 bis 2,0 M NaCl besitzt.

Die so behandelten Fraktionen werden mit dem Chromatographie-Material in Kontakt gebracht, so daß die Wirkstoffe an der Trägeroberfläche absorbiert werden. Vorzugsweise werden Salzlösungen enthaltend Alkalihalogenid, wie Natriumchlorid, Kaliumchlorid, Lithiumchlorid, etc. verwendet. Die Ionenstärke der Waschlösung entspricht in etwa einer Ionenstärke, wie sie eine 0,5 bis 2,0 M NaCl besitzt.

Das erfindungsgemäße Verfahren gewährleistet in überraschend einfacher Weise die Abreicherung oder Entfernung von Endotoxinen aus für den therapeutischen Einsatz vorgesehenen Wirkstoffe enthaltenden Präparaten, die gen- und/oder biotechnologisch aus natürlichen Quellen gewonnen werden. Überraschenderweise führt die Vorinkubation mit Salz/Detergenzien zur Abtrennung der Endotoxine ohne das Ausbeute und Reinheit der Plasmid DNA bei der sich anschließenden chromatographischen Reinigung negativ beeinflußt wird.

Die Erfindung wird anhand der folgenden Beispiele näher erläutert.

Die nachstehend abgekürzten Puffer P1, P2, P3, QBT, QC und QN besitzen die folgende Zusammensetzung:

| | |
|---|---|
| P1 | 10µg/ml RNAseA, 50 mM TrisHCl, 100 mM EDTA |
| P2 | 200 mM NaOH, 1 % SDS |
| P3 | 3 M KAc, pH 5,5 |
| QBT | 750 mM NaCl, 50 mM MOPS, 15 % Alkohol*, pH 7,0, 0,15 % Tricon® X 100 |
| QC | 1,0 M NaCl, 50 mM MOPS, 15 % Alkohol, pH 7,0 |
| QN | 1,6 M NaCl, 50 mM MOPS, 15 % Alkohol, pH 7,0 |
| Endoterin Removal Buffer | 750 mM NaCl/ 10% Triton® X 100/ 50 mM MOPS pH 7,0 |

| | |
|---|---|
| * Als Alkohole werden vorzugsweise Isopropanol oder Ethanol eingesetzt. | |

### Beispiel 1

Reinigung von 100 mg endotoxinfreier pUC18 DNA mit Hilfe der Anionenaustauscher-Chromatographie.

Eine 10 l Fermenter-Kultur des Plasmids pUC18 wird zentrifugiert und das daraus resultierende Bakterien-Pellet mit 500 ml Puffer P1 resuspendiert und die alkalische Lyse durch die Zugabe von jeweils 500 ml Puffer P2 und P3 durchgeführt. Zelltrümmer, genomische DNA und SDS Präzipitate (SDS = Natriumdodecylsulfat) werden unter Verwendung einer Filtrationseinheit, wie sie beispielsweise in der WO 95/21177 vorgeschlagen werden, abgetrennt.

Dem "cleared Lysate (cL)" wird nun 1/10 Volumen (150 ml) eines Endotoxin Removal Buffers zugegeben und mit dem cL gemischt. Das Gemisch wird für eine Stunde bei 4°C inkubiert und anschließend mittels einer Schlauchpumpe auf eine Anionenaustauschersäule von 4,4 cm Durchmesser und 50 cm Länge mit einer Flußrate von 4 ml/min gepumpt. Die Säule wurde zuvor mit 350 ml QBT Puffer (10 ml/min) äquilibriert. Die Säule wird mit 2,5 1 QC (15 ml/min) gewaschen. Die Plasmid DNA wird mit 400 ml QN Puffer (3 ml/min) eluiert und anschließend mit 0,7 vol. Isopropanol präzipitiert und anschließend mit 70 %igem Ethanol gewaschen.

Die Bestimmung des Endotoxin Gehaltes erfolgt mit Hilfe des BioWhittaker LAL Tests. Die gereinigte DNA kann direkt als genetische Vakzine in Muskel oder andere Gewebe injiziert werden.

Nach der Endotoxinabreicherung weist die DNA nur noch Endotoxinkontaminationen von < 50 E.U./mg DNA auf.

**Tabelle:**

| Endotoxingehalt in DNA-Präparationen vor und nach der Anwendung des erfindungsgemäß Verfahrens. | | |
|---|---|---|
| DNA Prep. | Endotoxingehalt in E.U./mg DNA | |
| | Vorher | Nachher |
| 1 | 2500 | 10 |
| 2 | 4200 | 17 |
| 3 | 3300 | 15 |
| 4 | 2900 | 15 |
| 5 | 3900 | 12 |

### Beispiel 2

Reinigung von 10 mg endotoxinfreier pBR322 DNA mit Hilfe der Anionenaustauscher-Chromatographie.

Eine 5 l Schüttelkultur des Plasmids pBR322 wird zentrifugiert und das hieraus resultierende Bakterien Pellet mit 125 ml Puffer P1 resuspendiert und die alkalische Lyse durch Zugabe von jeweils 125 ml Puffer P2 und P 3 durchgeführt. Zelltrümmer, genomische DNA und SDS Präzipitate werden durch Zentrifugation abgetrennt. Das Lysat wird anschließend über einen Faltenfilter geklärt.

Dem cL wird 1/10 Volumen (35 ml) eines Puffers bestehend aus 20 % NP40, 750 mM NaCl, 50 mM MOPS pH 7,0 zugegeben mit dem cL-Gemisch und für eine Stunde bei 4°C inkubiert. Die DNA wir dann wie folgt isoliert: Das vorbehandelte cL wird auf eine QIAGEN® tip 10.000 Antionenaustauscher-Säule gegeben. Nach dem Durchfluß des cL wird die Säule mit dem Puffer QC gewaschen und die DNA anschließend mit Hilfe von Puffer QF (1,25 M NaCl, 50 mM Tris/HCl, 15 % Ethanol, pH 8,5) eluiert.

Die so hergestellte DNA kann an rekombinante Adenoviruspartikel angekoppelt werden. Der erhältliche Adenovirus/DNA Komplex kann für in vivo bzw. ex-vivo Gentherapie verwendet werden.

Die Biomasse einer 20 l Fermenterkultur des Plasmids pUC19 wird unter Zugabe von jeweils 1 l der Puffer P1, P2, P3 lysiert und anschließend über eine in einer Säule angeordneten Diatomeenerdeschüttung filtriert. Dem filtrierten Lysat wird 20 g Ni-NTA modifiziertes Silicagel zugegeben und für 30 min. bei RT auf dem Schüttler inkubiert. Anschließend wird das Ni-NTA modifizierte Silicagel durch Zentrifugation abgetrennt und der Überstand über eine Anionenaustauscher-Chromatographiesäule aufgereinigt.

## Patentansprüche

1. Verfahren zur Abreicherung oder Entfernung von Endotoxinen aus für den therapeutischen Einsatz vorgesehenen Nucleinsäuren, Viruspartikeln, Adenoviren, AAV oder Retroviren enthaltenden Präparaten, die gen- und/oder biotechnologisch aus natürlichen Quellen gewonnen werden, durch Behandlung mit chromatographischem Material, wobei
- die natürlichen Quellen aufgeschlossen werden, die erhaltenen Fraktionen gegebenenfalls zentrifugiert, filtriert oder mit affinitätschromatographischen Methoden behandelt werden,
- die Fraktionen mit einer wäßrigen Salzlösung und Detergenzien vorinkubiert werden, mit Anionenaustauscher-Material behandelt werden und danach mit einer weiteren Salzlösung gewaschen werden und die Wirkstoffe von dem Anionenaustauscher eluiert werden, um dann in an sich bekannter Weise weiter aufgereinigt zu werden.

2. Verfahren nach Anspruch 1, wobei die in den Präparaten enthaltenen Nucleinsäuren, Plasmid-DNA, RNA, YACs, genomische DNA der Größen 6 bp bis 1000 kbp sind.

3. Verfahren nach Anspruch 1 und/oder 2, wobei die natürlichen Quellen Zellen, Zellorganellen, Gewebe oder Mikroorganismen sind.

4. Verfahren nach einem der Ansprüche 1 bis 3, wobei die natürlichen Quellen durch alkalische Lyse, Druck (French Press), Boiling Lyse, Lysozym, Detergenzien oder Kombinationen davon aufgeschlossen werden zu einem sogenannten "cleared Lysate".

5. Verfahren nach einem der Ansprüche 1 bis 4, wobei das Anionenaustauschermaterial eine Oberflächenladung der Ionenaustauschergruppen von 1 bis 4 µM/m² Trägeroberfläche entsprechend 0,5 bis 500 µM/ml aufweist.

6. Verfahren nach einem der Ansprüche 1 bis 5, wobei das Anionenaustauschermaterial auf polymerem Trägermaterial aufgebaut ist, wie Agarose, Acrylamid, Dextran, Silicagel oder Kombinationen davon.

7. Verfahren nach einem der Ansprüche 1 bis 6, wobei eine Vorinkubation des Cleared Lysates mit Salzlösungen einer Konzentration entsprechend einer 0,1 bis 2,0 M NaCl-Lösung durchgeführt wird, insbesondere Salzlösungen der Salze Natriumchlorid, Kaliumchlorid, Guanidiniumhydrochlorid, Natriumperchlorat und anderen chaotropen Salzen, und die Konzentration an nicht ionischen Detergenzien, wie Triton® X 114, Triton® X 100, NP 40, Tween® 20, Tween® 80, Syperonic® F-68 im Bereich von 0,1 % bis 30 % liegt.

8. Verfahren nach einem der Ansprüche 1 bis 7, wobei nach Behandlung des vorinkubierten "cleared Lysate" mit dem Anionenaustauscher-Material ein Waschschritt mit Salzlösungen enthaltend Alkalihalogenide, wie NaCl, KCl, Lithiumchlorid, etc. entsprechend einer Ionenstärke von 0,5 bis 2,0 M NaCl durchgeführt wird.

9. Verfahren nach einem der Ansprüche 1 bis 8, wobei im Anschluß an das in Anspruch 1 beschriebene Verfahren Nucleinsäure über anorganische Materialien, wie Silicagel, Diatomeenerde, Glas, Aluminiumoxid, Titanoxid, Hydroxylapatit oder über organische Materialien, wie Agarose, Dextran, Acrylamid, Polystyrolharze sowie Copolymere aus dem monomeren Baustein der genannten Polymere aufgereinigt werden.

10. Verwendung eines Anionenaustauschers gemäß Anspruch 5 in Kombination mit wäßriger Salzlösung und Detergenzien zur Abreicherung oder Entfernung von Endotoxinen aus nucleinsäurehaltigen Präparaten, die zum Einsatz in der Gen- und/oder Biotechnologie, insbesondere Gentherapie vorgesehen sind.

11. Wäßrige Lösung enthaltend Alkalihalogenide in Konzentrationen von 0,1 bis 2,0 M, nichtionische Tenside in Mengen von 0,1 bis 30 % sowie 10 bis 100 mM MOPS.

12. Zusammenstellung enthaltend die wäßrige Lösung gemäß Anspruch 11, endotoxinfreie Pufferlösungen sowie gegebenenfalls Chromatographie- und Filtermaterial.

13. Verfahren nach Anspruch 1, wobei das cleared Lysate mit NTA-Agarose, IDA-Agarose, Silica-NTA oder deren unvollständigen Syntheseprodukte vorinkubiert wird.

## Claims

1. A process for the depletion or removal of endotoxins from preparations designated for therapeutical use containing nucleic acids, virus particles, adenoviruses, AAVs or retroviruses, which are obtained from natural sources by genetic engineering and/or biotechnology, by treatment with chromatographic material wherein
- said natural sources are lysed, the fractions obtained are optionally centrifuged, filtrated or treated with affinity-chromatographical methods;
- said fractions are preincubated with an aqueous salt solution and detergents, treated with anion exchange material and then washed with another salt solution, and the active ingredients are eluted from the anion exchanger, followed by further purification in a per se known manner.

2. The process according to claim 1 wherein the nucleic acids contained in said preparations are plasmid DNA, RNA YACs, genomic DNA having a size of from 6 bp to 1000 kbp.

3. The process according to claims 1 and/or 2 wherein said natural sources are cells, cell organells, tissues or microorganisms.

4. The process according to any of claims 1 to 3 wherein said natural sources are lysed by alkaline lysis, pressure (French Press), boiling lysis, lysozyme, detergents or combinations thereof to give a so-called "cleared lysate".

5. The process according to any of claims 1 to 4 wherein said anion exchange material has a surface charge of the ion exchanging groups of from 1 to 4 µM/m² of the support surface area, corresponding to 0.5 to 500 µM/ml.

6. The process according to any of claims 1 to 5 wherein said anion exchange material is based on polymeric support material, such as agarose, acrylamide, dextrane, silica gel or combinations thereof.

7. The process according to any of claims 1 to 6 wherein a preincubation of the cleared lysate is performed with salt solutions having concentrations corresponding to 0.1 to 2.0 M NaCl solution, in particular salt solutions of the salts sodium chloride, potassium chloride, guanidinium chloride, sodium perchlorate, and other chaotropic salts, and the concentration of non-ionic detergents, such as Triton® X 114, Triton® X 100, NP 40, Tween® 20, Tween® 80, Syperonic® F-68, is in the range of from 0.1% to 30%.

8. The process according to any of claims 1 to 7 wherein said treatment of the preincubated "cleared lysate" with said anion exchange material is followed by a washing step with salt solutions containing alkali halides, such as NaCl, KCl, lithium chloride etc., corresponding to an ionic strength of 0.5 to 2.0 M NaCl.

9. The process according to any of claims 1 to 8 wherein the process as described in claim 1 is followed by a further purification of the nucleic acid by inorganic materials, such as silica gel, diatomaceous earth, glass, alumina, titania, hydroxyapatite, or by organic materials, such as agarose, dextrane, acrylamide,polystyrene resins and copolymers from the monomeric components of the mentioned polymers.

10. Use of an anion exchanger as defined in claim 5 in combination with an aqueous salt solution and detergents for the depletion or removal of endotoxins from preparations containing nucleic acids designated for use in genetic engineering and/or biotechnology, especially in gene therapy.

11. An aqueous solution containing alkali halides in concentrations of from 0.1 to 2.0 M, non-ionic surfactants in amounts of from 0.1 to 30%, and 10 to 100 mM MOPS.

12. A kit containing the aqueous solution according to claim 11, endotoxin-free buffer solutions, and optionally chromatographic and filtering material.

13. The process according to claim 1 wherein the cleared lysate is preincubated with NTA-agarose, IDA-agarose, silica-NTA or their incomplete synthesis products.

## Revendications

1. Procédé pour réduire ou éliminer les endotoxines de préparations contenant des acides nucléiques, des particules virales, des adénovirus, des virus associés aux adénovirus (AAV) ou des rétrovirus, prévues pour une utilisation thérapeutique, préparations qui sont obtenues par génie génétique et/ou par une biotechnologie à partir de sources naturelles, par traitement avec un matériau chromatographique, procédé dans lequel :
- les sources naturelles sont attaquées, les fractions obtenus sont éventuellement centrifugées, filtrées ou traitées par des techniques de chromatographie d'affinité,
- les fractions sont préincubées avec une solution aqueuse de sels et des détergents, traitées avec un matériau échangeur d'anions, puis lavées avec une autre solution de sels, et les principes actifs sont élués de l'échangeur d'anions, pour ensuite subir une purification plus poussée d'une manière connue en soi.

2. Procédé selon la revendication 1, dans lequel les acides nucléiques contenus dans les préparations sont un ADN plasmidique, un ARN, des chromosomes YAC, un ADN génomique, ayant une longueur de 6 bp à 1000 kbp.

3. Procédé selon la revendication 1 et/ou 2, dans lequel les sources naturelles sont des cellules, des organelles cellulaires, des tissus ou des micro-organismes.

4. Procédé selon l'une des revendications 1 à 3, dans lequel les sources naturelles sont attaquées par une lyse alcaline, une pression ("French Press"), une lyse par ébullition, un lysozyme, des détergents ou des combinaisons de ces derniers, pour donner ce que l'on appelle un "lysat clarifié".

5. Procédé selon l'une des revendications 1 à 4, dans lequel le matériau échangeur d'anions présente une charge superficielle des groupes échangeurs d'anions de 1 à 4 µM/m² de surface support, ce qui correspond à 0,5-500 µM/ml.

6. Procédé selon l'une des revendications 1 à 5, dans lequel le matériau échangeur d'anions est constitué d'un matériau support polymère tel que l'agarose, l'acrylamide, le dextrane, le gel de silice ou leurs combinaisons.

7. Procédé selon l'une des revendications 1 à 6, dans lequel on procède à une préincubation du lysat clarifié avec des solutions de sels ayant une concentration correspondant à une solution de NaCl 0,1 à 2,0 M, en particulier des solutions des sels chlorure de sodium, chlorure de potassium, chlorhydrate de guanidinium, perchlorate de sodium et autres sels chaotropes, et la concentration de détergents non-ioniques, tels que le Triton ®X 114, le Triton ®X 100, le NP 40, le Tween® 20, le Tween® 80, le Syperonic® F-68, est comprise entre 0,1 et 30 %.

8. Procédé selon l'une des revendications 1 à 7, dans lequel, après traitement du "lysat clarifié" préincubé avec le matériau échangeur d'anions, on procède à une étape de lavage avec des solutions de sels contenant des halogénures de métaux alcalins tels que NaCl, KCl, le chlorure de lithium, etc., correspondant à une force ionique de 0,5 à 2,0 M de NaCl.

9. Procédé selon l'une des revendications 1 à 8, dans lequel, après le procédé défini dans la revendication 1, on purifie l'acide nucléique sur des matériaux inorganiques tels que le gel de silice, la terre de diatomées, le verre, l'oxyde d'aluminium, l'oxyde de titane, l'hydroxylapatite, ou sur des matériaux organiques, tels que l'agarose, le dextrane, l'acrylamide, les résines de polystyrène, ainsi que les copolymères obtenus à partir du constituant monomère des polymères mentionnés.

10. Utilisation d'un échangeur d'anions selon la revendication 5 en combinaison avec une solution aqueuse de sels et des détergents pour réduire ou éliminer les endotoxines de préparations contenant des acides nucléiques, qui sont prévues pour être utilisées en génie génétique et/ou en biotechnologie, en particulier en thérapie génique.

11. Solution aqueuse contenant des halogénures de métaux alcalins en des concentrations de 0,1 à 2,0 M, des tensioactifs non-ioniques en des quantités de 0,1 à 30 %, ainsi que du MOPS 10 à 100 mM.

12. Composition contenant la solution aqueuse selon la revendication 11, des solutions tampons sans endotoxines, et éventuellement un matériau pour chromatographie et filtration.

13. Procédé selon la revendication 1, dans lequel le lysat clarifié est préincubé avec du NTA-agarose, de l'IDA-agarose, de la silice-NTA, ou leurs produits de synthèse incomplets.
